# EUROPEAN PATENT SPECIFICATION

(11) **EP 0 409 299 B1**
(45) Date of publication and mention of the grant of the patent: **07.08.1996**
(21) Application number: 90201704.5
(22) Date of filing: 27.06.1990
(51) Int. Cl.: C12N 9/28, C12N 15/56, C12N 1/20, C12N 1/21, A21D 8/04

(54) **Mutant enzyme having reduced stability under industrial application conditions**
Mutiertes Enzym mit reduzierter Stabilität unter industriellen Anwendungsbedingungen
Enzyme muté avec une stabilité réduite sous des conditions industrielles

(30) Priority: 29.06.1989 EP 89201732
(43) Date of publication of application: 23.01.1991
(73) Proprietor: GIST-BROCADES N.V., NL-2600 MA Delft (NL)
(72) Inventor: Van Eijk, Jan Henricus, NL-3722 AT Bilthoven (NL); Quax, Wilhelmus Johannes, NL-2253 VB Voorschoten (NL); Sanders, Johan Pieter Marinus, NL-2613 BV Delft (NL)
(74) Representative: Matulewicz, Emil Rudolf Antonius, Dr.

(56) References cited:
- EP-A- 0 028 491
- EP-A- 0 251 446
- EP-A- 0 273 268
- US-A- 4 416 903
- BIOLOGICAL ABSTRACTS, vol. 87, no. 7, April 1989, Philadelphia, PA (US); N.A. SMIRNOVA et al., AN 70127
- BIOLOGICAL ABSTRACTS, vol. 87, no. 7, April 1989, Philadelphia, PA (US); N.A. SMIRNOVA et al., AN 70128
- PROTEIN ENGINEERING, vol. 1, no. 3, June 1987, Eynsham, Oxford (GB); P. JOYET et al., p. 241
- JOURNAL OF THE BOLOGICAL CHEMISTRY, vol. 264, no. 32, 15 November 1989, American Society for Biochemistry & Molecular Biology Inc., Baltimore, MD (US); Y. SUZUKI et al., pp. 18933-18938
- PROTEIN ENGINEERING, vol. 3, no. 4, March 1990, Eynsham, Oxford (GB); N. DECLERCK et al., p. 354

## Description

### Technical field

The invention relates to a mutant enzyme having reduced stability and to a method for the production thereof.

### Background and Relevant Literature

Most flours used for bread making are supplemented at the mill or at the bakery with α-amylase. The prior art suggests that fungal and cereal α-amylase preparations can be used for improving loaf volume and that bacterial and cereal α-amylases have also a crumb softening effect. Studies on bread staling have indicated that the recrystallization of the starch fraction during storage of bread causes the increase in crumb firmness. Consequently a crumb softening effect can be obtained by partially degrading the starch fraction during the baking process. Effective antifirming action of α-amylase requires that the enzyme survives in the baking dough until a sufficient part of the starch fraction is gelatinized to permit the hydrolysis to occur (Miller et al., Food Technology, January 1953, p. 38). The low thermostability of fungal α-amylase produced by Aspergillus oryzae is such that this enzyme is largely inactivated by the time the starch in the baking product gelatinizes and may be attacked and hydrolyzed by the enzyme. Therefore fungal α-amylases will hardly improve crumb softness, although they can be used for improving loaf volume without any danger of causing overdextrination.

On the other hand, bacterial α-amylases are characterized by such a high thermostability that too much starch can be dextrinized during baking. If too much enzyme has been added, the bread crumb will be very gummy and sticky and the bread quality becomes unacceptable for the consumer. Bacterial amylases partly survive the baking process and will continue their action after baking especially during slow cooling of bread. In order to obtain an acceptable crumb softening effect it is necessary to control the dosage of bacterial α-amylase and the bread making process conditions very strictly. Because of the problems associated with the use of these thermostable enzymes, bacterial amylases are not generally used by the baking industry. α-Amylases of intermediate thermostability seem most suitable for improving crumb softness, because bread crumbs are less easily overdextrinized by high dosages of these enzymes. For this reason malt α-amylases which are characterized by a thermostability in between that of fungal and bacterial α-amylase, are still used extensively by the baking industry. However, the presence of proteolytic side activities in many malt preparations cause undesirable side effects. Furthermore a purified α-amylase prepared from malt is much too expensive for application in baking.

Other possibilities are given in U.S. patent 4,320,151, which describes a method for improving the low thermostability of fungal α-amylase during the baking process, and in European patent application EP-A-0273268 which describes a chemical modification method for decreasing the high thermostability of bacterial α-amylase. In both cases the enzymes have to be modified before use in the bread dough to obtain intermediate thermostability. Fungal α-amylase can be protected against thermal denaturation by solubilising or dispersing the enzyme in a protective medium according to the U.S. patent, whereas bacterial α-amylase has to be acylated before use in the dough according to the European patent application. Thus, there still is a need for new enzymes with an optimal stability, which are suitable for direct use in an industrial process such as the baking process.

### Summary of the invention

The present invention provides a modified bacterial α-amylase which is a product of a microbial fermentation process and exhibits reduced thermostability under baking conditions relative to the corresponding wild-type enzyme and having an amino sequence which differs in at least one amino acid from the wild-type α-amylase at the amino acid number 113, 114, 116, 123, 163, 164, 166, 238, 316, 322, 345, 349, 356, 386, 394 or 398 of α-amylase derived from B. amyloliquefaciens or a homologous position in a homologous α-amylase.

### Brief description of the Figures

### Figure 1: Structure of plasmid pUCAm4.

A 2.2 kb BglII-BamHI fragment carrying the α-amylase gene of Bacillus amyloliquefaciens was inserted into the BamHI site of pUC 18. The ampicillin resistance gene is indicated by AMP.

### Figure 2: DNA sequence of α-amylase gene.

The insert of pUCAm4 revealed a single large open reading frame of 1542 bases encoding α-amylase of Bacillus amyloliquefaciens. The amino acid sequence is shown in the single letter code.

### Figure 3: Sequence of the EcoRI-BamHI fragment of pMaTBac.

The EcoRI site at position 3753 corresponds to the EcoRI site in pMa 5-8 at position 3753. The TAC promoter is located between position 3753 and 3858. The α-amylase gene is shown by the amino acid sequence in the single letter code.

### Figure 4: Residual activity of wild-type (WT) and combination mutant α-amylase after incubation at 75°C during 10 minutes. Mutants are as in Table 7.

### Figure 5: Residual activity of wild-type (WT) and mutant α-amylase after incubation at 75°C during 10 minutes.

Mutants are different substitutions at residue 123, as depicted in Table 8.

### Figure 6: Chromosomal mapping of B. amyloliquefaciens H2 DNA, 5' and 3' integrants and the α-amylase negative strain BAM 112.

### Description of the Specific Embodiments

Enzymes with suitable stability can be developed or found in several ways, for example by classical screening methods or by using modern genetic and protein engineering techniques.

Screening for organisms or microorganisms that display the desired enzymatic activity, can be performed for example by isolating and purifying the enzyme from a microorganism or from a culture supernatant of such microorganisms, determining its biochemical properties and checking whether these biochemical properties meet the demands for a particular use. If the identified enzyme cannot be obtained from its natural producing organism, recombinant-DNA techniques may be used to isolate the gene encoding the enzyme, express the gene in another organism, isolate and purify the expressed enzyme and test whether it is suitable for the intended use.

Another way of obtaining new enzymes for an intended use is the modification of existing enzymes. This can be achieved inter alia by chemical modification methods (see I. Svendsen, Carlsberg Res. Commun. 44 (1976), 237-291). In general these methods are too unspecific in that they modify all accessible residues with common side chains, or they are dependent on the presence of suitable amino acids to be modified, and are often unable to modify amino acids which are difficult to reach, unless the enzyme molecule is unfolded.

Alternatively, enzyme modification through mutagenesis of the encoding gene does not suffer from the aspecificities mentioned above, and therefore is thought to be superior. Mutagenesis can be achieved either by random mutagenesis or by site-directed mutagenesis.

Random mutagenesis by treating whole microorganisms with chemical mutagens or with mutagenizing radiation may of course result in modified enzymes. In this case strong selection protocols to search for these particular, rare mutants have to be available. Higher probability of isolating mutant enzymes by random mutagenesis can be achieved, after cloning the encoding gene, by mutagenizing it in vitro or in vivo and expressing the encoded enzyme by recloning of the mutated gene in a suitable host cell. Suitable hosts for the production of the modified enzymes are, for example, bacteria (E. coli, Bacillus), yeasts or fungi (Aspergillus). Also in this case suitable biological selection protocols must be available in order to select the desired mutant enzymes. These biological selection protocols do not necessarily select directly the enzymes which are best suited for industrial application.

The present invention now provides novel mutant enzymes, which can be obtained by expression of a gene encoding said enzyme, having an amino acid sequence which differs in at least one amino acid from the corresponding wild-type enzyme, and which exhibits reduced stability during industrial application conditions. Although some mutant enzymes are known to be less stable under laboratory conditions, in industrial application conditions only a small change in stability was noticed due to difference in test and application conditions (temperature, pH, substrate, etc.). We have for the first time succeeded to obtain mutants which show reduced stability under industrial process conditions.

Site directed mutagenesis enabling specific substitution of one or more amino acids by any other desired amino acid can be used to construct and further select an enzyme with improved properties.

According to one aspect of the invention combinations of identified mutations can be made in order to modulate the desired stability characteristics precisely. Fine tuning of the stability of the mutant enzymes is possible by combining appropriate mutants.

All kinds of forms of stability of the enzyme can be altered, for example, temperature stability, pH-stability, stability during mixing or stability in the presence of chemical compounds like substrates, salts, inhibitors etc.

Mutagenesis of the encoding gene or fusion of encoding genes have already been applied as techniques for constructing α-amylase mutants having altered enzymatic properties. In a recent patent application (EP 0208491) a method is described for constructing bacterial α-amylase hybrid enzymes by constructing of the fusion α-amylase genes from B. licheniformis and B. stearothermophilus. N.A. Smirnova et al (Biological Abstracts, 87, no. 7 (1989) abstract no. 70127 and abstract no. 70128) have constructed B. amyloliquefaciens α-amylase mutants having reduced thermostability under laboratory test conditions. These tests conditions are quite different from the baking conditions, where the pH is rather low (pH = 5-5.5), whereas the starch content and the viscosity is very high. As will be shown in the reference Example, not all mutant α-amylases having a reduced thermostability under laboratory test conditions will have an optimal thermostability under baking application conditions. The present invention provides a method for the preparation of a mutant enzyme which exhibits a desired stability under industrial application conditions which is exemplified by the preparation of mutant baking enzymes by a procedure for selecting the mutants most suitable for application in bread making. By baking enzymes is meant enzymes involved or applied in dough making.

By a mutant enzyme is meant an enzyme which differs in one or more amino acids from the wild-type enzyme. The gene encoding the mutant enzyme has a DNA sequence which differs in at least one, preferably in 1-10, residues from the wild-type gene. The mutant enzyme is produced in a microbial fermentation process, whereafter the enzyme may be isolated or purified. The mutant enzyme according to the present invention exhibits the desired stability under industrial application conditions and can be used as such without further chemical modification. For example, the mutant enzyme may be an α-amylase which exhibits reduced thermostability under baking conditions. Other baking enzymes having amylolytic, hemicellulolytic, proteolytic, lipolytic or oxidoreductase activities can be applied for improving the quality of bakery products as well (see for example the review in AIB Technical Bulletin (1980) Vol. 11, 10, 11, 12). The properties of the existing enzymes like thermostability, pH optimum, substrate specificity, activity, etc. are not always optimal for their application as a baking enzyme. The present invention provides a method to optimize the properties of these existing enzymes, making them more suitable for application in bread making. Using protein engineering, enzymes can be constructed, which will have their highest activity during one of the phases of the bread making process. According to one aspect of the invention α-amylase is provided having exactly the optimal thermostability which corresponds to acting of the α-amylase mostly during and not after the baking phase of the bread making process. Another example of improving existing baking enzymes by protein engineering is the preparation of a mutant proteolytic enzyme, which becomes inactivated during the mixing phase of the bread making process and therefore will act as a mixing time reducer, which is active during mixing only.

The modified α-amylase will have an amino acid sequence which differs at least in one selected amino acid from the wild-type enzyme. The abovementioned mutations are obtained from the Bacillus amyloliquefaciens α-amylase as will be described in detail hereafter. For example bacterial α-amylase having an amino acid sequence which differs at least one amino acid from the wild type enzyme on the amino acid number 113, 114, 116, 123, 163, 164, 166, 238, 316, 322, 345, 349, 356, 386, 394 or 398 of B. amyioliquefaciens or a homologous position in a homologous α-amylase. Advantageously α-amylase with the mutation of the amino acid number 123 can be applied. The skilled person in the art will appreciate that suitable enzymes may be obtained as well with mutation of an amino acid corresponding to other parts of the amino acid sequence.

According to another aspect of the invention the amino sequence of the modified α-amylase has been modified on at least two amino acid numbers. This may result in an enzyme which shows an effect of reduced stability which is more than each of the contributions of the single mutations. In this way a mutant α-amylase can be prepared having an optimal stability.

Suitable hosts for the production of modified α-amylase are E. coli, Bacillus subtilis, Bacillus licheniformis and Bacillus amyloliquefaciens. Preferably the gene is integrated into the host.

The modified α-amylase shows improved properties during industrial application, for example, in the bread baking process. With improved properties as used in the specification in connection with a mutant α-amylase, we mean reduced thermostability during baking, relative to the corresponding wild type enzyme.

According to this invention a mutant enzyme can be designed based on a careful examination of the structure of wild type enzyme, combined with careful biochemical investigation of the stability of the original enzymes, for example the thermostability of bacterial α-amylase under baking conditions, followed by a rational modification of the wild type gene sequence. Extensive investigation of designed mutants under industrial application conditions has resulted in the identification of mutants with optimal properties.

According to one aspect of the invention the mutant α-amylase with reduced thermostability during application, for example in the baking process, can be used in a dough or bread (or related products) making process. By related products is meant the products originating from baking a batter or dough from a mixture of water and ground cereal meal.

### Materials and Methods

### 1. General cloning techniques

Cloning techniques have been used as described in the handbooks of T. Maniatis et al., 1982, Molecular Cloning, Cold Spring Harbor Laboratory; F.M. Ausubel et al., 1987, Current Protocols in Molecular Biology, John Wiley & Sons Inc., New York; B. Perbal, 1988, A practical Guide to Molecular Cloning, 2nd edition, John Wiley & Sons Inc., New York. These handbooks describe in detail the protocols for construction and propagation of recombinant DNA molecules, the procedures for making gene libraries, the procedures for sequencing and mutating DNA and the protocols for the enzymatic handling of DNA molecules.

### 2. Chemical mutagenesis

Cloned DNA may be treated in vitro with chemicals in order to introduce mutations in the DNA. If these mutations are directed to amino acid encoding triplet codons a mutated protein can be produced by the mutated cloned DNA. A method for chemical mutagenesis with the aid of sodium bisulfite is described by Shortle and Botstein (Methods Enzymol., 1983, 100, 457). A preferable method is described by Folk and Hofstetter (Cell, 1983, 33, 585). Other methods for mutagenesis are described by Smith, Ann. Rev. Genet., 1985, 19, 423. A particular useful protocol is described by Ausubel et al., ibid. (see chapter 8).

### 3. Mutagenesis on gapped-duplex DNA

A method based on the gapped-duplex approach (Kramer et al., 1984, Nucl. Acids Res. 12, 9441) and a phasmid (plasmid/phage hybrid) was used. Essentially the method rests on a gapped duplex DNA intermediate consisting of a gapped strand (-strand) containing a wild-type antibiotic resistance marker and a template strand (+ strand) carrying an amber mutation in the gene conferring resistance to the antibiotic. After annealing, the mutagenic oligonucleotide becomes incorporated in the gapped strand during in vitro gap-filling and sealing reaction. The resultant molecules are used to transform a mismatch repair deficient (Mut S) host in which the linkage between the intended mutation and the antibiotic resistance marker is preserved. The mixed phasmid population, isolated from this strain, is then allowed to segregate in a suppressor negative host strain. Transformants are plated on antibiotic containing medium, thus imposing a selection for progeny derived from the gapped strand.

The twin vector system pMa/c 5-8, which was described by P. Stanssens et al. (in Protein Engineering and Site-directed Mutagenesis, 1985, 24th Harden Conference, Programme and Abstracts, A.R. Fersht and G. Winter eds.) is composed of the followina elements:
- pos 11-105:: bacteriophage fd, terminator
- pos 121-215:: bacteriophage fd, terminator
- pos 221-307:: plasmid pBR322 (pos 2069-2153)
- pos 313-768:: bacteriophage f1, origin of replication (pos 5482-5943)
- pos 772-2571:: plasmid pBR322, origin of replication and β-lactamase gene
- pos 2572-2685:: transposon Tn903
- pos 2719-2772:: tryptophan terminator (double)
- pos 2773-3729:: transposon Tn9, chloramphenicol acetyl transferase gene
- pos 3730-3803:: multiple cloning site

The sequence is published (Stanssens et al, 1987, EMBO-course, Martinsried; Stanssens et al, 1989, Nucleic Acids Res., 17, 4441-4454).

In the pMa type vector nucleotide 3409 is changed from G to A, while in the pMc type vector nucleotide 2238 is changed from G to C, creating amber stopcodons in the acetyl transferase gene and β-lactamase gene, respectively, rendering said genes inactive.

All sequences referred to were obtained from Genbank™, National Nucleic Acid Sequence Data Bank, NIH USA. Plasmid pMc 5-8 has been deposited (DSM 4566). To perform mutagenesis the target DNA fragment is cloned into the multiple cloning site of pMa 5-8. Subsequently a gapped duplex between pMa 5-8 containing the target DNA and pMc 5-8 is constructed.

The single strand gap, consisting of the target DNA, can be subjected to mutagenesis with a mutagenic oligonucleotide, with long synthetic oligonucleotides with a low level of misincorporated nucleotides, with chemicals or with enzymatic misincorporation of nucleotides. For a detailed description see Ausubel et al., ibid. or Perbal, ibid. As an alternative to in vitro mutagenesis one can use in vivo mutagenesis either with the aid of UV-light or chemicals or by the application of an E. coli mutator strain (Fowler et al., J. Bacteriol. 1986, 167, p. 130).

Mutagenic nucleotides can be synthesised using apparatus obtainable from Applied Bio Systems.

### 4. Random mutagenesis by enzymatic misincorporation of nucleotides

A pMa/pMc gapped duplex can be subjected to primer extension and misincorporation mutagenesis as originally described by Shortle et al., 1982, Proc. Nat. Acad. of Science 79, p. 1588-1592, and by Cunningham and Wells (Prot. Eng., 1987, 1, p. 319) or preferably by a modification of the procedure as described by Lehtovaara et al., (Prot. Eng., 1988, 2, p. 63).

This method is based on controlled use of polymerases. Four populations of DNA molecules are first generated by primer elongation of a gapped duplex of pMa/pmc so that they terminate randomly in the gap but always just before a known type of base (before A, C, G or T, respectively). Each of four populations is then mutagenized in a separate misincorporation reaction where the correct base can now be omitted. In this way all types of base substitution mutations can be generated at every position of the gap. As a modification on Lethovaara we used Sequenase™ (United States Biochemical Corporation, Cleveland, OH) instead of Klenow DNA polymerase and MoMuLV reverse transcriptase (BRL) instead of AMV-reverse transcriptase. We also observed that the use of higher reverse transcriptase concentrations resulted frequently in multiple consecutive mutations. This can be considered as advantageous in order to obtain labile enzymes.

On the other hand to ensure single site substitutions we have introduced the following modification to the protocol described by Lehtovaara et al., ibid. In the reverse transcriptase buffer not three but only one misincorporating nucleotide is present. For instance the A-specific limited base elongation mixture is incubated in three separate reactions with 250 µM dCTP, 250 µM dGTP and 250 µM dTTP respectively. For a complete set of 4 base specific limited elongation mixture a total set of 12 separate misincorporation reactions is carried out. After 1.5 hour incubation at 42°C a chase of all four deoxynucleotides in a concentration of 0.5 mM is added and the reactions are further incubated for at least 20 minutes at 37°C. Samples are then further processed according to Lehtovaara et al. (ibid.) with the modification that no counterselection to an uracil-containing DNA strand but a counterselection based on the pMa/c vector was applied.

### 5. Isolation of mutant α-amylase

E. coli WK6 cells harbouring plasmid pMaTBac are grown in BHI-medium containing the appropriate selection agent. 10 ml of an overnight culture is spinned down and resolved in 1 ml 20% sucrose, 1 mM EDTA. After 15 minutes of incubation at 20°C cells are spinned down again. The celpellet is then resuspended in 1 ml MilliQ H₂O and kept at 0°C during 10 minutes. After spinning down the sferoblasts, supernatant harbouring the α-amylase is adapted to 2 mM CaCl₂, 0.7 mM MgCl₂ and 2.5 mM NaHCO₃. Whenever necessary, the α-amylase can be purified by conventional biochemical methods.

### 6. α-Amylase activity

α-Amylase activities were routinely determined using Phadebas™ tablets from Pharmacia. In this method the solubilization of dye labelled starch by α-amylase in a buffer pH=5.5 during 15 minutes at 30°C is measured spectrofotometrically. α-Amylase activity is expressed in Phadebas Units (PU) using an Aspergillus oryzae fungal α-amylase preparation of 10.000 PU/g as an internal standard. One Phadebas Unit defined this way equals about 10 SKB units, used in the baking industry.

Residual α-amylase activity in bread crumb was determined using a slightly different procedure. A bread crumb suspension was prepared from 10 g bread crumb and ml buffer pH=5.5 using a Waring blender at full speed during one minute. 0.1-1.0 ml of the bread crumb suspension was incubated overnight (18 hours) at 30°C in a Phadebas test. Residual activity in the bread crumb was calculated by comparing the activity of the heat-treated amylase in the bread crumb suspension to the activity of the untreated enzyme, added to a bread crumb suspension of a control bread, prepared without bacterial α-amylase.

### 7. Testing thermolability of α-amylase in application (baking) test

The thermolability of selected α-amylase mutants was tested in a puploaf baking test. Puploaves were baked from 150 g dough pieces obtained by mixing 200 g flour (100%), 110 ml water (55%), 3 mg ascorbic acid (15 ppm), 1.4 g instant dry yeast (0.7% Fermipan™), 4 g salt (2%), 3 g sugar (1.5%), 1 g shortening (0.5%), 400 mg CaCl₂.2H₂O (0.2%), 10 mg fungal α-amylase P₂₀₀ (2250 SKB/kg flour) and variable amounts of wild type and mutant bacterial α-amylase. After mixing for 6 minutes and 15 seconds at 52 r.p.m. in a pin mixer, the dough was divided, proofed for 45 minutes at 30°C, punched, proofed for another 25 minutes, moulded and panned. After a final proof of 70 minutes at 30°C the dough was baked for 20 minutes in an oven at 240°C and loaf volume was determined by the rapeseed displacement method. A Stevens Texture Analyzer was used to determine the crumb softness of 2 slices from the center of the puploaves, which had been stored in plastic bags for 72 hours at room temperature. Crumb firmness values are expressed as the force (g) required for compressing a 2 cm thick slice of bread for 5 mm (25%) using a probe of 1.0 inch diameter and a rate of compression of 0.5 mm/sec. The thermolability of the various bacterial α-amylase samples was expressed as the number of α-amylase units (PU) required for obtaining an optimal crumb softening effect without causing overdextrination (20-30% reduction of firmness values).

### 8. Evaluation of baking performance of bacterial α-amylase mutants

### 1. Loaf volume improving effect

The loaf volume improving effect of (mutant) bacterial α-amylase was determined in the puploaf bread making procedure described above, except that CaCl₂, fungal α-amylase and shortening have been omitted in the recipe.

### 2. Crumb softening effect

A dough was prepared from 3500 g flour (100%), 1960 ml water (55%), 87.5 g compressed yeast (2.5%), 52.5 g sugar (1.5%), 70 g salt (2%), 210 mg fungal α-amylase P₂₀₀ (2700 SKB/kg flour), 17.5 g shortening (0.5%), 105 mg ascorbic acid (30 ppm), 94.5 mg cysteine (27.5 ppm) and variable amounts of wild type or mutant bacterial α-amylase. After mixing in a Kemper spiral mixer (350 rotations at speed 1, followed by 1200 rotations at speed 2), 900 g dough pieces were rounded, proofed for 35 minutes at 30°C, punched, moulded, panned, proofed for 65 minutes at 34°C and baked for 30 minutes in an oven at 220°C. Loaf volume was determined by displacement of rapeseed and the gummyness of the bread crumb was judged by a consumer panel into 4 categories. (0: crumb not gummy, not overdextrinized; 0/+: crumb not gummy, slightly dextrinized (optimal performance); +: crumb slightly gummy, slightly overdextrinized; +++: crumb very gummy, severely overdextrinized). For measuring crumb firmness 2 slices of 2 cm thickness from the center of the loaf were analyzed by a Stevens Texture Analyzer, using a probe of 1.5 inch diameter, a compression depth of 5 mm (25%) and a rate of compression of 0.5 mm/sec.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity and understanding, it will be readily apparent to those of ordinary skill in the art in light of the teachings of this invention that certain changes and modifications may be made thereto without departing from the spirit and scope of the appended claims.

The following examples further illustrate the invention.

### Example 1

### Molecular cloning of the Bacillus amyloliquefaciens α-amylase gene

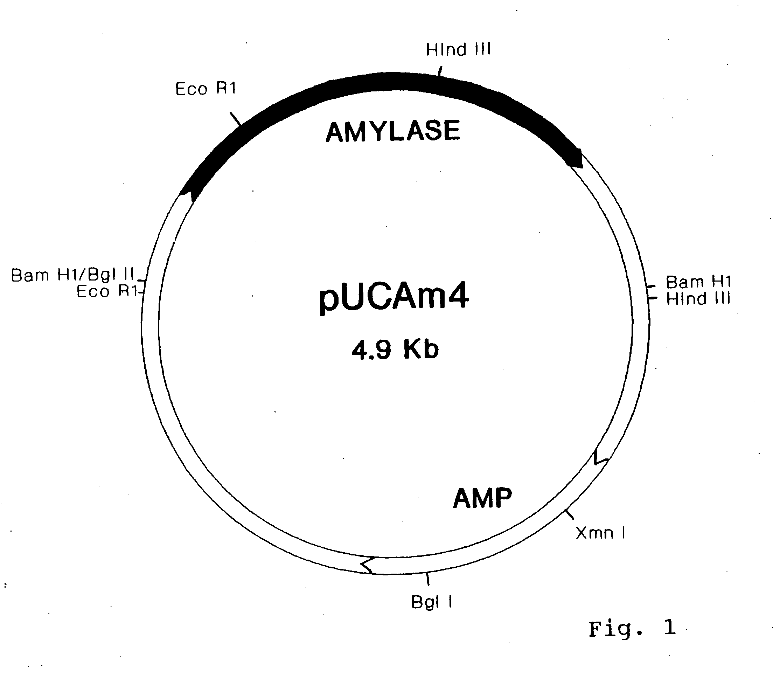

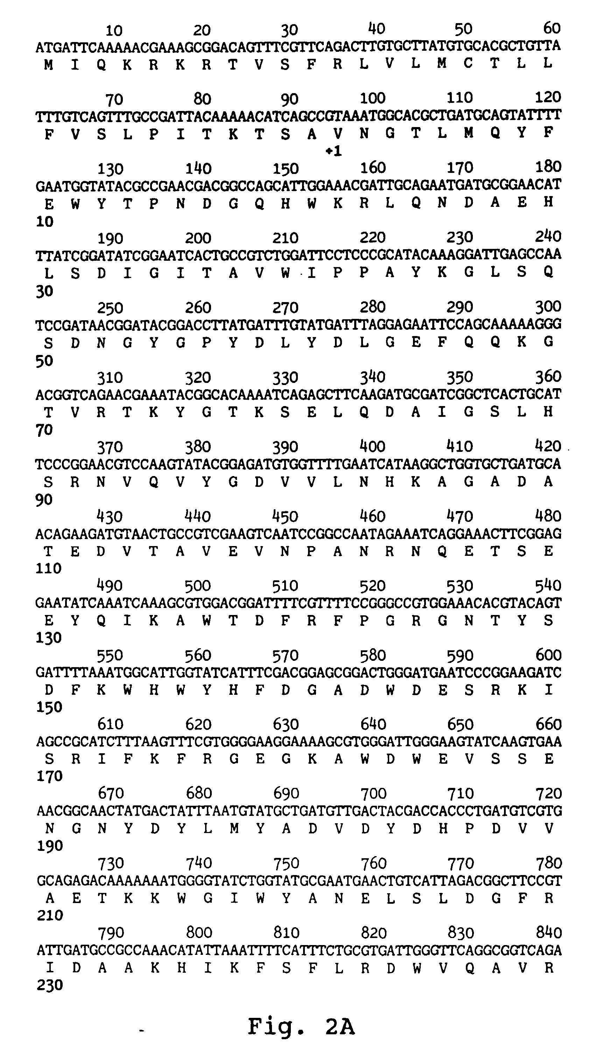

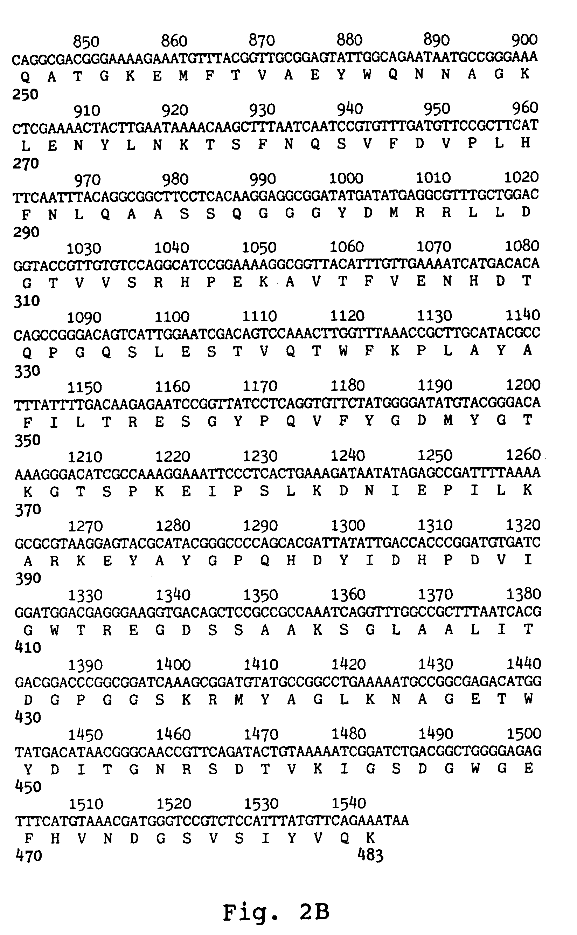

Chromosomal DNA isolated from Bacillus amyloliquefaciens H2, a derivative of Bacillus strain H IAM1521 (Hartley, 1968, Biochemistry 7, 2401-2408) was digested with restriction enzyme BclI and ligated into the BclI site of plasmid pUN121 (Nilsson et al., 1983, Nucleic Acids Res. 11, 8019). This plasmid carries an ampicillin resistance gene, a tetracyclin resistance gene and a c1-repressor gene. Transcription of the tetracyclin gene is prevented by the gene product of the c1-repressor gene. Insertion of foreign DNA into the unique BclI site of the c1-repressor gene results in activation of the tetracyclin resistance. This allows positive selection of recombinants on ampicillin/tetracyclin containing agar plates. The ligation mixture was transformed into E. coli HB101 (ATCC 33694). Ampicillin/tetracyclin resistant colonies were tested for α-amylase production on LB-plates (Ausubel, ibid.) supplemented with 0.4 g/l starch acc. Zulkowsky (Merck). After growth and incubation with I₂ vapor, a positive E. coli colony producing a large clearing halo was selected for further characterization. The matching plasmid, pUNH2, was shown to contain a 5.5 kb BclI-Bcl1 insert originating from Bacillus amyloliquefaciens H₂. A 2.2 BglII-BaMH1 fragment was isolated from pUNH2 using Gene Clean (obtainable from B10 101, La Jolla, LA, USA) and ligated into the BamHI site of pUC18 (Pharmacia). The resulting plasmid, pUCAm4 is shown in Figure 1. The insert of pUCAm4 was sequenced by the method of Sanger (Proc. Natl. Acad. Sci. USA, 1977, 74, 6463). The DNA sequence revealed an open reading frame of 1542 bases (Figure 2) encoding a signal sequence of 31 amino acids plus a mature protein of 483 amino acids. The sequence of the protein was identical to the α-amylase sequence of Bacillus amyloliquefaciens as determined by Takkinen et al. (J. Biol. Chem., 1983, 258, 1007).

### Example 2

### Construction of mutagenesis/expression vector pMaTBac

A 2.2 kb SmaI-BamHI fragment was derived from pUCAm4 and purified by the Gene Clean method. This fragment was ligated into SmaI-BamHI digested pMc5-8. The resulting plasmid pMcAm was obtained by transformation of E. coli WK6 (CBS 473.88) (Zell, R. and Fritz H.J., EMBO J., 1987, 6, p. 1809). After infection with phage M13K07 single stranded pMcAm was isolated as described by the supplier (Pharmacia). Double stranded plasmid pMa5-8 was digested with SmaI and BamHI and annealed with single stranded pMcAm in order to obtain a gapped duplex (Kramer et al., Nucleic Acids Res., 1984, 12, 9441). This gapped duplex was subjected to site directed mutagenesis (Kramer et al., ibid., Zell, R. and Fritz H.J.,ibid.) with an oligonucleotide designed to introduce a XbaI site at position -18 relative to the startcodon. The sequence of this oligonucleotide is:

5'-TTC CTC TCC CTC TAG ATT TCT TAT AC.

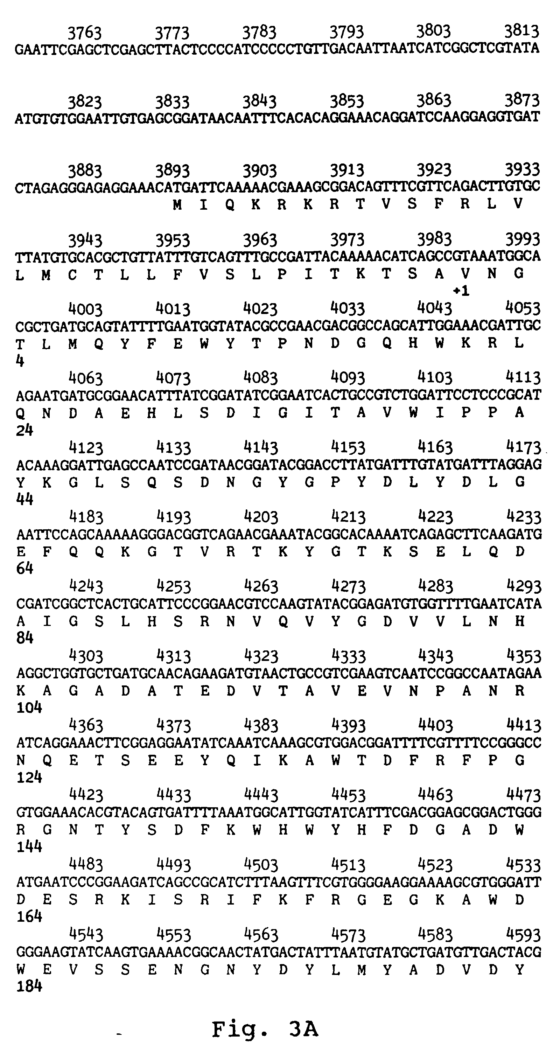

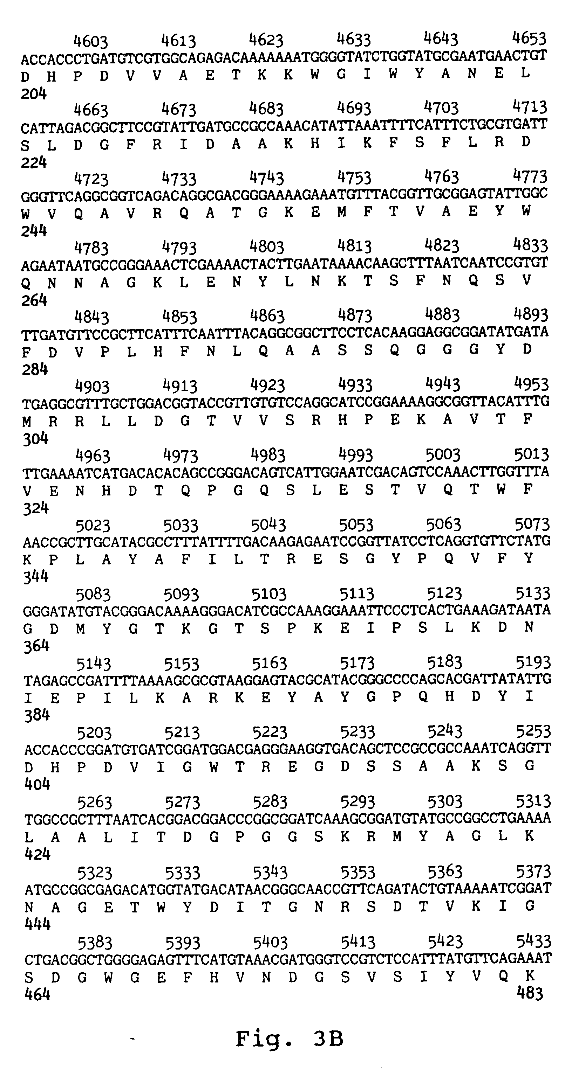

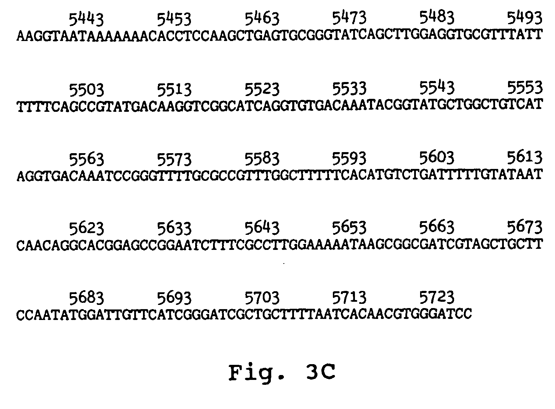

After successful mutagenesis the plasmid was passed through a DAM E. coli strain (E. coli GM48, Phabagen, Utrecht) and then the EcoRI-XbaI fragment was removed by partial digestion and substituted by a synthetic EcoRI-XbaI fragment carrying a copy of a tac promoter. The sequence of this fragment is depicted together with the α-amylase encoding insert of the resulting pMaTBac (CBS 287.89) in Figure 3. This plasmid gives rise to the synthesis of α-amylase in E. coli under guidance of the IPTG inducible tac promoter. Furthermore the insert of this vector can be subjected to mutagenesis by making suitable gapped duplex molecules between pMa and pMc type DNA molecules. Single stranded DNA for the respective molecules can be obtained by infection of E. coli WK6 with phage M13K07 as described by the supplier (Pharmacia).

### Example 3

### Bisulphite mutagenesis of pMaTBac

Single stranded pMaTBac was annealed with KpnI-ApaI digested pMcTBac in order to obtain a heteroduplex with a gap running from position 4915 to 5146 (see Figure 3A-C). This heteroduplex was subjected to bisulphite mutagenesis (see Experimental). After transformation into E. coli WK6 Muts (CBS 472.88) (Zell, R. and Fritz H.J., ibid.) and selection on chloramphenicol containing agar plates (25 µg/ml) plasmid pools were isolated and transformed into E. coli WK6. Resulting transformants were grown in BHI-medium (DIFCO) containing 2.0 mM CaCl₂, 25 µg/ml chloroamphenicol and 0.15 mM IPTG (SIGMA) during 24 hours at 37.C. Three samples of the supernatant were incubated during 0, 7 and 14 minutes at 80°C, respectively, and then assayed for α-amylase activity. This was done by spotting the samples (5-10 µl) on 0.2% starch containing BHI plates, incubating for 1 hour at 55°C and coloring the plates with an I₂ solution (3 g I₂/l and 7 g KI/l). Colonies, which showed a decrease of halo size and intensity relative to wild-type colonies as a result of prolonged incubation at 80°C, were selected as heat-labile α-amylase mutants.

Table 1 shows the sequence of the mutations obtained in a typical experiment. DNA sequences were determined by sequencing the gap region after isolation of single stranded DNA of pMcTBac mutants. Mutant pMcTbac plasmids were grown in BHI medium in strain E. coli WK6 and enzyme preparations were made by the osmo-chock method (Ausubel et al., ibid., Materials and Methods) to release periplasmic α-amylase.

Table 2 shows the optimal dosage of the mutant α-amylases and the baking results.

As loaf volume can differ from one baking experiment to another all comparisons are made relative to a control enzyme preparation (WT) which is included in every separate experiment. This WT enzyme is prepared by fermenting non-mutagenized plasmid pMaTBac in E. coli WK6.

**Table 1**

| OBTAINED THERMOSENSITIVE α-AMYLASE MUTANTS AFTER BISULFITE MUTAGENESIS | | | | | |
|---|---|---|---|---|---|
| MUTANT | AMINO ACID (AA) no | FROM → TO | | AA → AA | |
| A8 | 394 | CCG | TCG | Pro | Ser |
| | 386 | TAC | TAT | Tyr | Tyr |
| B10 | 345 | CCG | TCG | Pro | Ser |
| E12 | 398 | CCC | TCC | Pro | Ser |
| G2 | 386 | CCG | CTG | Pro | Leu |
| 3 | 349 | GCC | GCT | Ala | Ala |
| | 322 | ACA | ATA | Thr | Ile |
| 4 | 345 | CCG | TCG | Pro | Ser |
| 6 | 322 | ACA | ATA | Thr | Ile |
| | 316 | CAT | TAT | His | Tyr |
| 7 | 356 | TCC | TTC | Ser | Phe |

**Table 2.**

| BAKING TESTS WITH BISULFITE MUTANT α-AMYLASES | | |
|---|---|---|
| MUTANT | OPTIMAL DOSAGE | LOAF VOLUME |
| | (PU/200 g FLOUR) | (ml) |
| WT (PARENT STRAIN) | 4.25 | 563 |
| A8 | 14 | 574 |
| B10 | 9 | 596 |
| E12 | 14 | 577 |
| G2 | 8.5 | 591 |
| 3 | 7.8 | 526 |
| 4 | 9.6 | 557 |
| 6 | 6.4 | 543 |
| 7 | 11.1 | 550 |

### Example 4

### Mutagenesis of pMaTBac by enzymatic misincorporation

Single stranded pMaTBac (see Example 2) was annealed with EcoRV-KpnI digested pMcTBac in order to obtain a heteroduplex with a gap running from position 4018 to 4915 (see Figure 3). The gapped duplex was subjected to enzymatic misincorporation mutagenesis as described in the experimental section.

A sample obtained after A-limited primer elongation was split in three parts and incubated in the presence of reverse transcriptase with dCTP, dGTP and dTTP, respectively. After incubation at 37°C for 10 minutes a chase with all four dNTP's and klenov polymerase and T4-DNA ligase was given to finish the elongation to completely double stranded molecules. These molecules were transformed into E. coli WK6 Muts and plasmid pools were recovered. These plasmid pools were transformed into E. coli WK6 and the colonies were selected on chloramphenicol (25 µg/ml) containing agar plates.

Resulting mutants were screened for thermosensitive α-amylase by the method as described in Example 3.

Table 3 shows the sequence of several thermolabile α-amylase obtained in a typical experiment. DNA sequences were determined by sequencing the gap after isolation of single stranded DNA of pMcTBac mutants.

Table 4 shows the optimal dosage of the mutant α-amylases and the baking results.

**Table 3**

| OBTAINED THERMOSENSITIVE AMYLASE MUTANTS AFTER LIMITED ELONGATION | | | | | |
|---|---|---|---|---|---|
| A-limited | | | | | |
| MUTANT | AA no. | FROM → TO | | AA → AA | |
| 12 | 116 | GTC | GAC | VAL | ASP |
| 13 | 113 | GTA | GGA | VAL | GLY |
| | 114 | ACT | ACC | THR | THR |
| | 116 | GTC | GCC | VAL | ALA |
| 14 | 163 | TGG | CGG | TRP | ARG |
| | 164 | GAT | GAG | ASP | GLU |
| | 166 | TCC | CCC | SER | PRO |
| 17 | 238 | TTT | CTT | PHE | LEU |
| 25 | 116 | GTC | GCC | VAL | ALA |
| 26 | 116 | GTC | GGC | VAL | GLY |
| 29 | 113 | GTA | GCA | VAL | ALA |
| | 114 | ACT | ACG | THR | THR |
| | 116 | GTC | GCC | VAL | ALA |
| T-limited 15 | 123 | AGA | TGT | ARG | CYS |

**Table 4**

| BAKING TESTS WITH α-AMYLASE OBTAINED BY A OR T-LIMITED ELONGATION | | |
|---|---|---|
| MUTANT | OPTIMAL DOSAGE | LOAF VOLUME |
| | (PU/200 g FLOUR) | (ml) |
| WT (PARENT STRAIN) | 4.4 | 566 |
| 12 | 13.4 | 525 |
| 13 | 19.5 | 564 |
| 14 | 11 | 542 |
| 15 | > 100 | 560 |
| 17 | 4.25 | 545 |
| 25 | 5.4 | 558 |
| 26 | 12.5 | 561 |
| 29 | 14.6 | 558 |

### Example 5

### Comparison of wild type and mutant B. amyloliquefaciens α-amylase in bread making

In Table 5 the loaf volume improving effect in puploaves is compared for the fungal, the wild type bacterial and the mutant no. 15 B. amyloliquefaciens α-amylase.

From the results it appears that the number of α-amylase units required for obtaining maximal loaf volume is very similar (≈ 50 PU/200 g flour) for the fungal, the wild type B. amyloliquefaciens and the mutant no. 15 B. amyloliquefaciens α-amylase. When the fungal or the bacterial mutant no. 15 α-amylase is used a maximal increase in loaf volume of about 15% is obtained without causing any overdextrination of the bread crumb. It is not possible, however, to obtain the maximal loaf volume without causing severe overdextrination, when using the B. amyloliquefaciens wild type α-amylase for improving loaf volume.

In Table 6 the crumb softening effect of the wild type and the bacterial mutant no. 15 (Arg 123 → Cys 123) α-amylase are compared in a standard bread making recipe containing 2700 SKB of fungal α-amylase per kg flour.

From Table 6 it appears that the wild type bacterial α-amylase is a very efficient crumb softener. An optimal crumb softening effect is already obtained at dosages between 3.9-13 PU/kg flour. However, a severe overdextrination of the bread crumb is already observed at a slightly higher dosage of 39 PU/kg flour. A much higher dosage of about 74 PU/kg flour is required of the mutant enzyme to obtain an optimal crumb softening effect, and dosage of 740 PU/kg may be added before a slight overdextrination of the bread crumb is observed. Thus the danger of overdextrination is considerably reduced when using the mutant no. 15 instead of the wild type B. amyloliquefaciens α-amylase as a crumb softener.

The reduced thermostability of the bacterial mutant no. 15 α-amylase is also evident from the residual amylase activity in the bread crumb. Whereas the wild type bacterial α-amylase largely survives the baking process (50-75% residual activity), no residual α-amylase activity could be detected in bread prepared from doughs containing the bacterial mutant no. 15 α-amylase.

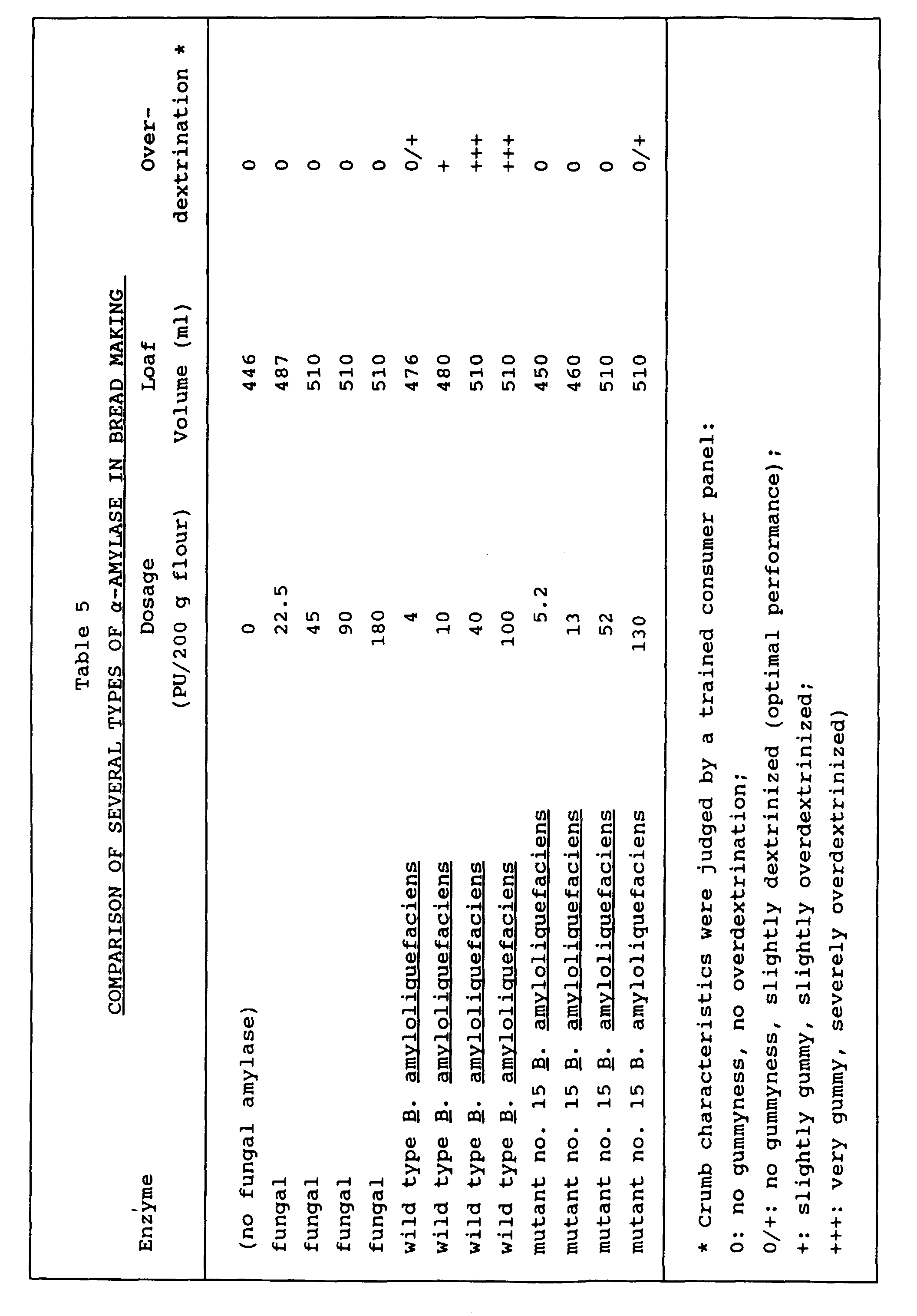

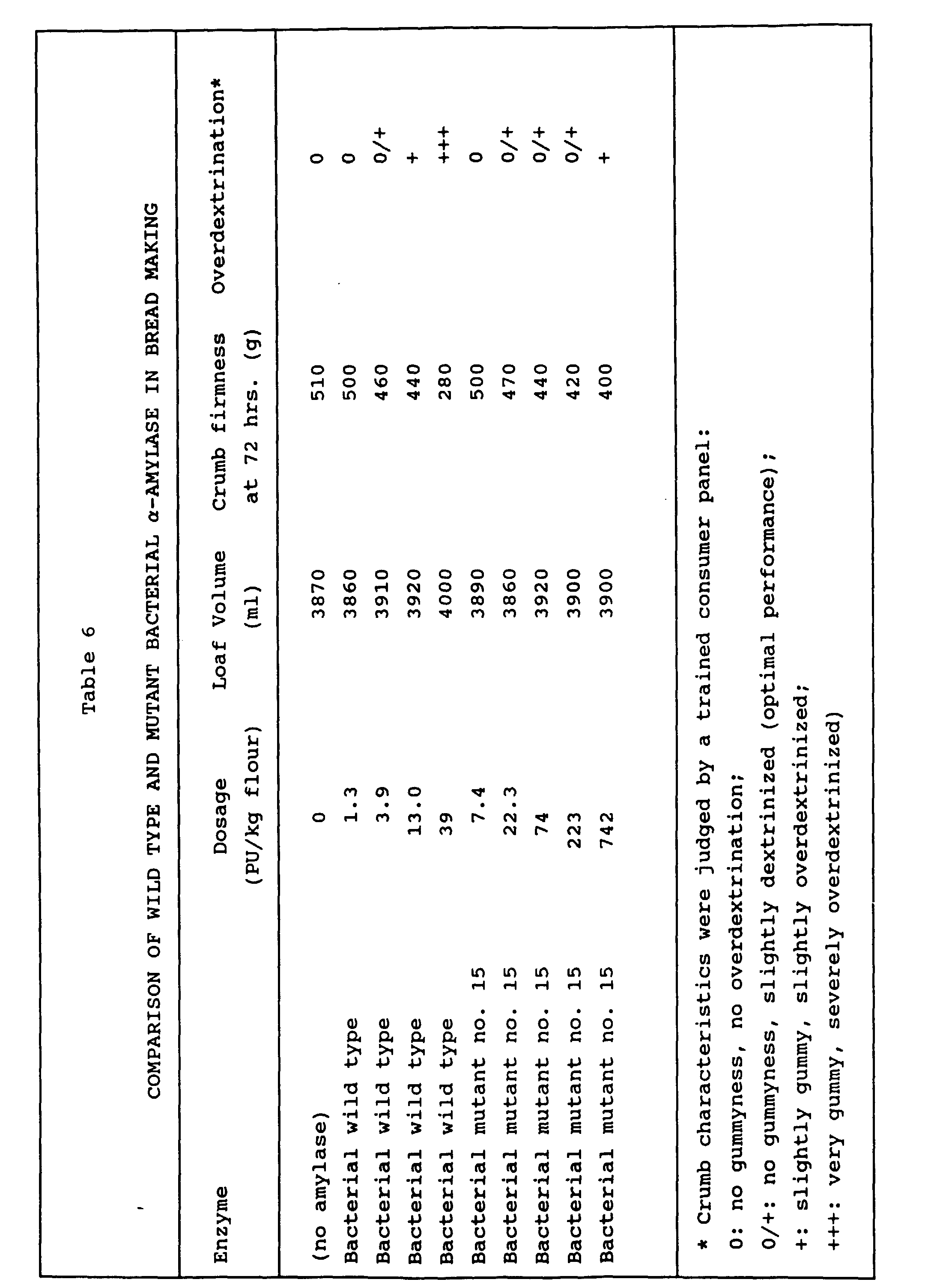

### Example 6

### Modulation of thermolability of α-amylases by combining different single mutations

Several of the mutants described in Example 3 and Example 4 were found to retain too high stability for optimal usefulness in bread making. Since the individual amino acid substitutions of each mutant α-amylase are known a design of a mutant with an optimal stability/lability for application in bread can be done by combining individual amino acid substitutions. The latter can be done by exchanging appropriate restriction fragments of mutated pMaTBac or by introducing the amino acid substitution under consideration by site-directed mutagenesis (Material and Methods, section 3). Table 7 shows a number of combinations which have been made. These combination mutants were tested on stability by incubating supernatant samples at 75°C during 10 minutes and testing on remaining α-amylase activity using BHI starch plates and incubation during 1 hour at 55°C (see Example 3).

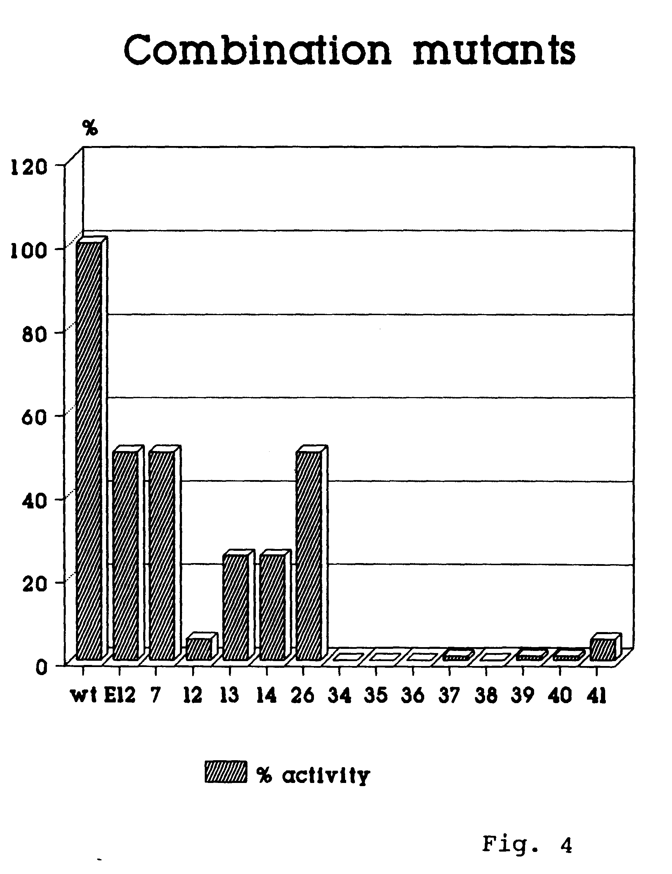

By comparison with appropriate dilutions of non-heated WT and mutant α-amylase the remaining activity percentage was determined (Figure 4). It can be seen that all the combination mutants were more thermolabile than the corresponding parent mutants. Fine tuning of thermolability therefore can be obtained by combining appropriate single site mutants.

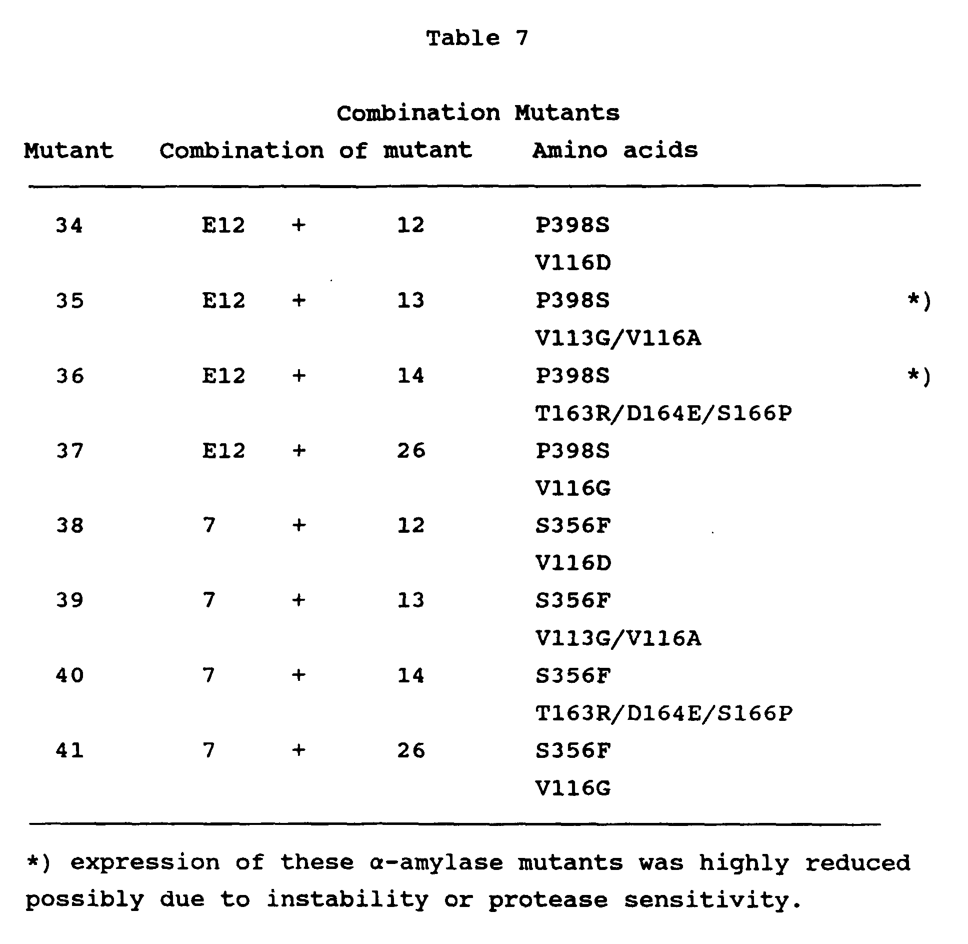

### Example 7

### Modulation of thermolability by substituting different amino acids at selected positions

The methods applied in Example 3 and Example 4 have identified residue positions that are important for stability/lability of α-amylase. However, due to the specific nature of the mutagenesis method only a limited number of substitutions can be expected at a certain position. In order to broaden the range of mutations and corresponding stability/lability behaviour one can substitute a residue, which was found to be important for stability/lability for all possible natural amino acids. This can be done by site directed mutagenesis using an oligonucleotide with a mixture of all four possible nucleotides at the codon in consideration (see Materials and Methods, section 3). With this aim a mixed oligonucleotide of the following sequence 
               C
CGAAGTTTCCTGATT NNATTGGCCGGATTGAC
               G
 was used to obtain a number of different α-amylases mutated in residue 123. N represents any of the 4 possible nucleotides (Table 8).

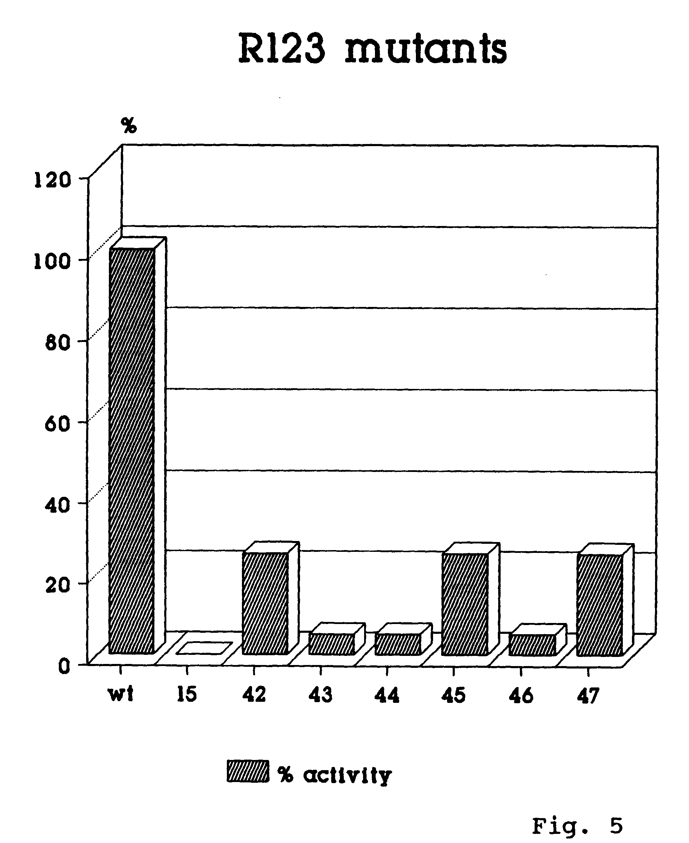

By the same method as in Example 6 the remaining acitivity percentage was determined (Figure 5). It can be seen that all mutants at position 123 studied are less stable than wild-type enzyme (R123). Furthermore it is clear that different amino acids at position 123 have different thermolability behaviour. Therefore a random mutagenesis at positions selected by the method of Example 3 and 4 can be used to select or design a mutant α-amylase with the desired stability/lability behaviour.

**Table 8**

| Random mutagenesis at a single residue position | |
|---|---|
| Mutant | Amino Acid Substitution |
| 15 | R123C |
| 42 | R123H |
| 43 | R123V |
| 44 | R123L |
| 45 | R123A |
| 46 | R123P |
| 47 | R123D |

### Example 8

### Replacement of chromosomal wild-type α-amylase gene by a mutant gene

To produce a mutant α-amylase recloning in an expression host can be chosen for. A preferred host may be the parent microorganism of the α-amylase under consideration. However, prior to use the endogeneous α-amylase gene has to be inactivated or deleted. This example describes the deletion of the α-amylase gene of B. amyloliquefaciens. Subsequently the mutant α-amylase gene can be expressed from a plasmid (e.g. pUB110) carrying the gene or from a chromosomally integrated copy of the gene. The latter is preferred for production purposes because no heterologous DNA is present in the host.

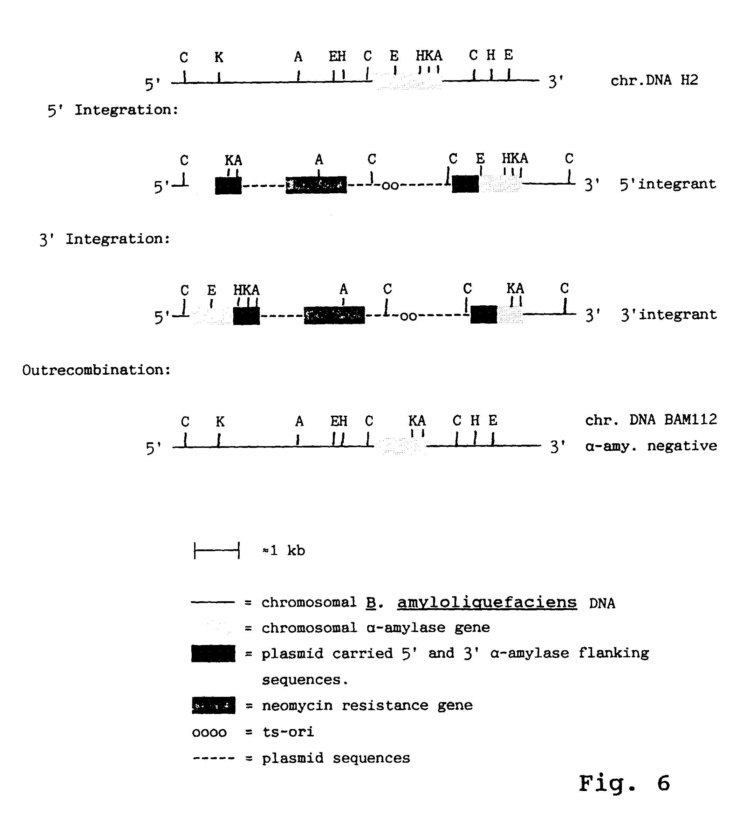

Inactivation of the chromosomal α-amylase of B. amyloliquefaciens was achieved by integration of a plasmid (the pE194 based pE194neo, see EP 0283075) containing a temperature sensitive origin of replication into the gene. Selection for chromosomal integrants was performed by increasing the temperature to nonpermissive conditions for plasmid replication in the presence of the relevant antibiotic. The plasmid contained a copy of the α-amylase gene with the middle part deleted (see Figure 6). Recombination can occur at both flanking sequences. Only if integration and excision occur by recombination at two different flanking sequences, this will result in the eventual loss of the α-amylase gene. These potential integrants were grown at 37°C in the absence of neomycin for stimulating the outrecombination and curing of the plasmid. Three α-amylase negative clones were obtained, one of which was extensively analyzed by chromosomal mapping. Also the parent-integrant of the α-amylase negative clone was analyzed by chromosomal mapping. In Figure 6 the restriction maps of chromosomal integration and the excision are shown. An α-amylase negative B. amyloliquefaciens strain was obtained and named BAM112. This strain had a deletion of 735 bp on the internal amylase gene from EcoRV to HindIII.

The α-amylase negative strain BAM112 was subsequently used for the production of mutant α-amylases. First the encoding DNA of mutant 15 (R123C) was cloned into pE194neo and transformed into strain BAM112 using protoplast transformation (Chang and Cohen, 1979, Molecular and General Genetics, 168, 111-115). Chromosomal integration of the mutated α-amylase gene (R123C) was achieved after selection at 50°C and 20 µg/ml neomycin. A number of integrants were isolated and after recombination occurred at both flanking sequences it was possible to select for integration of the 123C α-amylase gene. In a similar way as shown in Figure 6 excision of the plasmid from the chromosome was obtained. An α-amylase positive, neomycin sensitive clone, BAM115, was selected and analyzed by chromosomal mapping.

### Example 9 (Reference example)

The B. amyloliquefaciens α-amylase mutants TS141 and TS191, described by N.A. Smirnova et al (Biological Abstracts, 87, no. 7 (1989) abstract no. 70127 and abstract no. 70128) have been tested both under laboratory and application conditions. The reduced thermostability of these mutants was confirmed using the plate test method described in Example 3. When comparing the activity of the TS141 mutant and the wild-type enzyme at both pH 5.5 and 6.5 by the Phadebas method it appeared that the TS141 mutant α-amylase was normally active at pH 6.5 but had lost nearly all its activity at pH 5.5. For this reason the TS141 (Asp¹¹⁴ → Asn¹¹⁴) mutant α-amylase is not suitable for baking application. The thermostability of the TS191 (Glu¹⁹¹ → Lys¹⁹¹) mutant α-amylase in bread making was judged from the dosage required for an optimal crumb softening effect. It appeared that the thermostability of this mutant enzyme under application conditions was very similar to the thermostability of the wild-type enzyme. Therefore this mutant does not exhibit a useful phenotype.

## Claims

1. A modified bacterial α-amylase which is a product of a microbial fermentation process and exhibits reduced thermostability under baking conditions relative to the corresponding wild-type enzyme and having an amino sequence which differs in at least one amino acid from the wild-type α-amylase at the amino acid number 113, 114, 116, 123, 163, 164, 166, 238, 316, 322, 345, 349, 356, 386, 394 or 398 of D α-amylase derived from B. amyloliquefaciens or a homologous position in a homologous α-amylase.

2. A modified bacterial α-amylase according to claim 1 which has an amino acid sequence whereby the Arg 123 of the wild-type enzyme is replaced by Cys 123.

3. A modified enzyme according to claim 1 or 2 obtained by expression of a gene encoding said modified enzyme having an amino acid sequence which differs in 1 to 10 amino acids from the wild-type α-amylase.

4. A process for producing a dough or similar product which comprises the use of a modified bacterial α-amylase according to claim 1.

5. A dough or similar product comprising a modified bacterial α-amylase with bread improving properties according to claim 1.

6. A process for producing bread or related product which comprises the inclusion in a dough of a modified bacterial α-amylase according to claim 1.

7. A microorganism capable of producing a modified bacterial α-amylase according to any one of claims 1-3.

8. A microorganism, preferably a bacterium, yeast or fungus, more preferably an E. coli, a Bacillus or an Aspergillus, preferably Bacillus subtilis, Bacillus amyloliquefaciens or Bacillus licheniformis, which comprises a gene encoding a modified bacterial α-amylase according to any one of claims 1-3.

9. A gene encoding the modified bacterial α-amylase according to any one of claims 1-3 having a DNA sequence which differs in 1-10 residues from the wild-type gene.

10. A vector or plasmid comprising a gene according to claim 9.

11. A microorganism transformed with a vector or plasmid according to claim 10.

12. A process for the production of a modified bacterial α-amylase according to any one of claims 1-3 which comprises the fermentation of a microorganism according to claim 7, 8 or 11 and optionally separating or purifying the formed mutant α-amylase.

13. A bread improver composition which comprises as an active ingredient a modified bacterial α-amylase with bread improving properties according to any one of claims 1-3.

## Patentansprüche

1. Veränderte bakterielle α-Amylase, die ein Produkt eines mikrobiellen Fermentationsvorgangs ist, im Vergleich zum entsprechenden Wildtypenzym eine verringerte Thermostabilität unter Backbedingungen aufweist und deren Aminosäuresequenz sich in mindestens einer Aminosäure an der Aminosäurenummer 113, 114, 116, 123, 163, 164, 166, 238, 316, 322, 345, 349, 356, 386, 394 oder 398 von der von B. amyloliquefaciens hergeleiteten Wildtyp-α-Amylase oder an einer homologen Stelle von einer homologen α-Amylase unterscheidet.

2. Veränderte bakterielle α-Amylase gemäss Anspruch 1, in der gegenüber der Aminosäuresequenz des Wildtypenzyms Arg 123 durch Cys 123 ersetzt ist.

3. Verändertes Enzym gemäss Anspruch 1 oder 2, das durch Expression eines besagtes modifizerte Enzym kodierenden Genes erhältlich ist und das eine Aminosäuresequenz aufweist, die sich in 1 bis 10 Aminosäuren von der Wildtyp-α-Amylase unterscheidet.

4. Verfahren zur Herstellung eines Teiges oder eines ähnlichen Produktes, dadurch gekennzeichnet, dass eine modifizierte bakterielle α-Amylase gemäss Anspruch 1 verwendet wird.

5. Teig oder ähnliches Produkt,enthaltend eine modifizierte bakterielle α-Amylase mit brotverbessernden Eigenschaften gemäss Anspruch 1.

6. Verfahren zur Herstellung eines Brotes oder eines verwandten Produktes, dadurch gekennzeichnet, dass eine modifizierte bakterielle α-Amylase gemäss Anspruch 1 einem Teig beigefügt wird.

7. Mikroorganismus, der eine modifizierte α-Amylase gemäss einem der Ansprüche 1 bis 3 herzustellen vermag.

8. Mikroorganismus, bevorzugt ein Bakterium, eine Hefe oder ein Pilz, besonders bevorzugt ein E. coli, ein Bacillus oder ein Aspergillus, darunter bevorzugt Bacillus subtilis, Bacillus amyloliquefaciens oder Bacillus licheniformis, dadurch gekennzeichnet, dass er ein Gen beinhaltet, das eine modifizierte bakterielle α-Amylase gemäss einem der Ansprüche 1 bis 3 kodiert.

9. Gen, welches die modifizierte bakterielle α-Amylase gemäss einem der Ansprüche 1 bis 3 kodiert und dessen DNA-Sequenz sich in 1 bis 10 Resten vom Wildtypgen unterscheidet.

10. Vektor oder Plasmid, ein Gen gemäss Anspruch 9 enthaltend.

11. Mikroorganismus, welcher mit einem Vektor oder plasmid gemäss Anspruch 10 transformiert ist.

12. Verfahren zur Herstellung einer modifizierten bakteriellen α-Amylase gemäss einem der Ansprüche 1 bis 3, dadurch gekennzeichnet, dass ein Mikroorganismus gemäss Anspruch 7, 8 oder 11 fermentieren gelassen und gewünschtenfalls die gebildete α-Amylasemutante abgetrennt oder gereinigt wird.

13. Backhilfsmittelzubereitung, welche als aktive Zutat eine modifizierte bakterielle α-Amylase mit brotverbessernden Eigenschaften gemäss einem der Ansprüche 1 bis 3 beinhaltet.

## Revendications

1. α-Amylase bactérienne modifiée qui est un produit d'un procédé de fermentation microbienne et qui présente une thermostabilité réduite dans des conditions de cuisson par rapport à l'enzyme de type sauvage correspondante et ayant une séquence en acide aminé qui diffère par au moins un acide aminé de l'α-amylase de type sauvage, à l'acide aminé numéro 113, 114, 116, 123, 163, 164, 166, 238, 316, 322, 345, 349, 356, 386, 394 ou 398 d'une α-amylase dérivée de B. amyloliquefaciens ou à une position homologue dans une α-amylase homologue.

2. α-Amylase bactérienne modifiée suivant la revendication 1, qui a une séquence en acide aminé dans laquelle l'Arg 123 de l'enzyme de type sauvage est remplacée par une Cys 123.

3. Enzyme modifiée suivant la revendication 1 ou 2, obtenue par expression d'un gène codant l'enzyme modifiée ayant une séquence en acide aminé qui diffère par 1 à 10 acides aminés par rapport à l'α-amylase de type sauvage.

4. Procédé de production d'une pâte ou d'un produit similaire, qui comprend l'utilisation d'une α-amylase bactérienne modifiée suivant la revendication 1.

5. Pâte ou produit similaire comprenant une α-amylase bactérienne modifiée à propriétés améliorant le pain, suivant la revendication 1.

6. Procédé de production de pain ou d'un produit semblable, qui comprend l'incorporation dans une pâte d'une α-amylase bactérienne modifiée suivant la revendication 1.

7. Micro-organisme capable de produire une α-amylase bactérienne modifiée suivant l'une quelconque des revendications 1 à 3.

8. Micro-organisme, de préférence une bactérie, une levure ou un champignon, avec avantage E. coli, un Bacillus ou un Aspergillus, de préférence Bacillus subtilis, Bacillus amyloliquefaciens ou bacillus licheniformis, qui comprend un gène codant une α-amylase bactérienne modifiée suivant l'une quelconque des revendications 1 à 3.

9. Gène codant l'α-amylase bactérienne modifiée suivant l'une quelconque des revendications 1 à 3, ayant une séquence d'ADN qui diffère par 1 à 10 résidus du gène de type sauvage.

10. Vecteur ou plasmide comprenant un gène suivant la revendication 9.

11. Micro-organisme transformé avec un vecteur ou plasmide suivant la revendication 10.

12. Procédé de production d'une α-amylase bactérienne modifiée suivant l'une quelconque des revendications 1 à 3, qui comprend la fermentation d'un micro-organisme suivant la revendication 7, 8 ou 11 et facultativement, la séparation ou la purification de l'α-amylase mutante formée.

13. Composition d'amélioration du pain, qui comprend comme ingrédient actif, une α-amylase bactérienne modifiée suivant l'une quelconque des revendications 1 à 3.
